Europäisches Patentamt

European Patent Office

Office européen des brevets

(11) Publication number: 0 309 047

A1

# (12) EUROPEAN PATENT APPLICATION

(21) Application number: 88202020.9

(22) Date of filing: 14.09.88

(51) Int. Cl.4: C07C 31/04 , C07C 29/15 , C07C 29/136 , C07C 69/04 , C07C 67/36 , B01J 31/04

(30) Priority: 24.09.87 GB 8722461

(43) Date of publication of application: 29.03.89 Bulletin 89/13

(84) Designated Contracting States: BE DE FR GB IT NL

(71) Applicant: SHELL INTERNATIONALE RESEARCH MAATSCHAPPIJ B.V. Carel van Bylandtlaan 30 NL-2596 HR Den Haag(NL)

(72) Inventor: Sie, Swan Tiong Badhuisweg 3 NL-1031 CM Amsterdam(NL) Inventor: Drent, Eit Badhuisweg 3 NL-1031 CM Amsterdam(NL) Inventor: Jager, Willem Wabe Badhuisweg 3 NL-1031 CM Amsterdam(NL)

(74) Representative: Aalbers, Onno et al P.O. Box 302 NL-2501 CH The Hague(NL)

(54) Process for the production of methanol and catalyst composition for said process.

(57) A process for the production of methanol comprising contacting a gaseous mixture comprising carbon monoxide and hydrogen with a catalytic system, obtainable by combination of:

component (a): a nickel salt,

component (b): an in situ alcohol providing agent, which originates from an external source, and

component (c): an alcoholate derived from an alkali metal or from an alkaline earth metal,

and activating the combined components, and a catalyst system to be used in this process.

## PROCESS FOR THE PRODUCTION OF METHANOL AND CATALYST COMPOSITION FOR SAID PROCESS

The invention relates to a process for the production of methanol and a catalyst composition for said process.

More particularly the invention relates to a process for the production of methanol by reaction of carbon monoxide with hydrogen in the presence of a catalytic system derived from at least a nickel salt.

A process for the production of methanol is described in US patent specification 4,619,946 comprising reacting at relatively low temperature carbon monoxide with hydrogen in the presence of a catalytic system derived from sodium hydride, sodium alcoholate and acetate of nickel, palladium or cobalt. The alcoholate applied is preferably a lower alkanolate having 1-6 carbon atoms and more preferably a tert-alkanolate, while as metal salt nickel acetate is preferably used.

The catalyst is subjected to a conditioning or activating step for a prolonged time with a gaseous mixture comprising carbon monoxide and hydrogen at such an elevated temperature and elevated pressure that a substantial amount of carbon monoxide and hydrogen is consumed for this conditioning.

Also in US patent specification 4,614,749 a process is disclosed for the production of methanol at relatively low temperature by reaction of carbon monoxide with hydrogen in the presence of a slurry catalyst system resulting from combination of
- a complex reducing agent comprising sodium hydridealcohol and an acetate of nickel, palladium or cobalt, and
- a carbonyl complex of one of the group VI metals.

The alcohol to be applied is preferably selected from alkanols having from 1 to 6 carbon atoms and more preferably tertiary amyl alcohol.

Another process for the production of methanol is described in published Japanese patent application No. 56-169,634. This process comprises reacting carbon monoxide and hydrogen in the presence of a catalyst comprising a nickel compound and a metal alkoxide, in the liquid phase at temperatures of 200 °C or lower. More preferably an alkali metal alkoxide might be used. The catalyst to be used for this process may be prepared by mixing a nickel compound with an alkali metal alkoxide, while it is preferred to use an organic diluent which is liquid under the preparation and use conditions of the catalyst system.

More particularly the teachings of this Japanese patent application instruct a person skilled in the art, that a high reaction rate may be reached by preparing the catalyst system with the use of a substantially alcohol free organic diluent and that it is desirable that an alcohol be not present in the reaction system at the commencement of the reaction.

Moreover from this Japanese patent application and especially from its example 2, it clearly appears that at low temperature only small amounts of methanol are produced in favour of production of methyl formate in large amounts.

Although improvements in the performances of the catalyst systems as described hereinbefore, could be reached as compared to those used in the conventional methanol manufacturing processes, requiring severe conditions, the still growing demand for cheaper methanol as starting material for a still increasing area of chemical syntheses evoked continuing research efforts for a further improved methanol manufacturing process as compared to the currently operated high pressure processes.

With the term improved methanol manufacturing process is meant a process utilizing a catalyst having enhanced activity at low temperatures, and retaining its activity for a long time under economically more attractive operating conditions.

An object of the present invention is therefore to provide such an improved manufacturing process for methanol. Another object of the present invention is to provide an improved catalytic system therefor.

As a result of extensive research and experimentation such a process was now surprisingly found. The process comprises contacting a gaseous mixture of carbon monoxide and hydrogen with a catalytic system, obtainable by combining the following components:

component (a): a nickel salt,

component (b): an in situ alcohol providing agent which originates from an external source, and

component (c): an alcoholate derived from an alkali metal or from an alkaline earth metal,

and activating the combined components.

The anion of the salt in component (a) may be derived from a great variety of acids. It is preferred that the salt in component (a) is a salt of a carboxylic acid or sulphonic acid. Among these acids preference is given to alkanoic acids having 1-10 carbon atoms in the chain or to paratoluene sulphonic acid. More preferably formic acid, acetic acid and oxalic acid or p-toluene sulphonic acid are used.

Examples of carboxylic acids from which component (a) also may be derived are dicarboxylic acids such as malonic acid, dimethylmalonic acid, succinic acid, glutaric acid, adipic acid, pimelic acid, suberic acid, azelaic acid, phthalic acid, isophthalic acid and terephthalic acid. The carboxylic acids from which component (a) may be derived may contain substituents, for example alkoxy groups, particularly those having not more than five carbon atoms, hydroxy groups, cyano groups and fluorine, chlorine, bromine and iodine atoms. Examples of such carboxylic acids are glycolic acid, 2-hydroxypropionic acid, 3-hydroxypropionic acid, glyceric acid, tartronic acid, malic acid, tartaric acid, tropic acid, benzilic acid, salicylic acid, anisic acid, gallic acid, 3,5-dichlorobenzoic acid, 3,5-dibromobenzoic acid, cyanoacetic acid, monofluroacetic acid, difluoroacetic acid, trifluoroacetic acid and trichloroacetic acid.

Other examples of suitable acids from which component (a) may be derived are propanoic acid, butanoic acid, 2-methylpropanoic acid, pentanoic acid, 3-methylbutanoic acid, 2,2-dimethylpropanoic acid, hexanoic acid, heptanoic acid and octanoic acid, hydrochloric acid, sulphuric acid, nitric acid and phosphoric acid.

A mixture of the salts in question may be used in component (a), for example formate and oxalate, formate and acetate, or acetate and oxalate.

The salts in component (a) may contain crystal water, but are preferably free therefrom.

The alcohol providing agent as component (b) may be any compound which may form in situ an alcohol under the activation conditions and/or reaction conditions of the methanol synthesis.

Preferably alkyl formates, alkyl oxalates or alkyl carbonates are applied or mixtures thereof. More preferably formates or oxalates are applied which are containing alkyl groups having 1 to 20 carbon atoms, particularly 1 to 10 carbon atoms, such as methyl, ethyl, propyl, butyl, isobutyl, tert-butyl, tert-amyl or 2-methyl-2-dodecyl. Most preferably methyl formate is used, providing in situ methanol under the reaction conditions.

The alcoholate of component (c) is preferably a sodium alcoholate or a potassium alcoholate. Among the alcoholates preference is given to alkoxides, particularly to those having in the range of from 1 to 20 carbon atoms per molecule, such as sodium methoxide, sodium ethoxide, sodium propoxide, sodium butoxide, sodium isobutoxide, sodium tert-pentoxide and potassium 2-methyldodec-2-oxide. Most preferred is potassium tert-butoxide.

The process according to the present invention may be carried out at a temperature and a pressure which are not critical and may vary within wide ranges. Preferably, a temperature in the range of from 30 °C to 120 °C and a pressure in the range of from 5 to 100 bar are used.

The process according to the present invention is usually carried out with an organic diluent in which the catalytic system is dissolved or is suspended. However also an excess of one of the components of the catalyst system e.g. formate and more preferably one having a higher boiling point, may be used as reaction medium. Suitably, a weight ratio of organic diluent to component (a) in the range of from 0.1 to 5000 is used, but this weight ratio may be lower than 0.1 or higher than 5000.

Examples of suitable diluents are ethers such as anisole, 2,5,8-trioxanonane (also referred to as "diglyme"), diethyl ether, diphenyl ether, diisopropyl ether and tetrahydrofuran; aromatic hydrocarbons, such as benzene, toluene, the three xylenes and ethyl-benzene; halogenated aromatic compounds, such as chlorobenzene and o-dichlorobenzene; halogenated alkanes, such as dichloromethane and carbontetrachloride; alkanes, such as hexane, heptane, octane, 2,2,3-trimethylpentane and kerosene fractions; cycloalkanes, such as cyclohexane and methylcyclohexane; sulphones, such as diisopropyl sulphone, tetrahydrothiophene 1,1-dioxide (also referred to as "sulfolane"), 2-methyl-4-butylsulfolane and 3-methylsulfolane. Mixtures of two or more solvents may be used. Very good results have been obtained with ethers and the use of diglyme is most preferred. The process may be carried out using a molar ratio of component (a) to component (b) which is not critical and may vary within wide ranges, preferably in the range of from 1:0.2 to 1:20 and more preferably of from 1:3 to 1:8.

The carbon monoxide and hydrogen may be used as pure gases or diluted with an inert gas such as a noble gas or nitrogen. The process according to the present invention may be carried out using a molar ratio carbon monoxide to hydrogen in the gaseous mixture which is not critical and may vary within wide ranges, suitably in the range of from 1:0.2 to 1:20. The carbon monoxide and hydrogen may preferably be obtained by partial oxidation of hydrocarbons, for example of natural gas with air or coal gasification, resulting in carbon monoxide-hydrogen mixtures containing nitrogen.

It will be appreciated by persons skilled in the art that the attractive results obtained according to the process of the present invention, i.e. significantly improved reaction rate and selectivity as to methanol at low temperatures, could certainly not be expected by them on account of the before mentioned prior art literature and that the teachings of more particularly the Japanese application 56-169,634 could only led away them from the present process.

The methanol produced according to the invention forms another feature of the invention. It may be used for a variety of purposes, for example for the manufacture of synthetic gasoline, as a fuel component and for the production of methyl tert-butyl ether.

The process according to the present invention may be carried out batchwise, semi-continuously or continuously.

It is preferred to remove methanol in the gaseous phase from the reaction mixture. This can be done by stripping the reaction mixture with carbon monoxide and hydrogen. Methanol can be recovered from the used stripping gas in any suitable manner for example by condensation.

It will be appreciated that another object of the present invention is formed by the catalyst system in the process as described hereinbefore and obtainable by combining the following components:
component (a): a nickel salt,
component (b): an in situ alcohol providing agent, which originates from an external source, and
component (c): an alcoholate derived from an alkali metal or from an alkaline earth metal.

It will be appreciated that another object of the invention is formed by the before identified catalyst system, mixed with one or more inert diluents and or carbon monoxide and hydrogen under an operational pressure suitable for the process of the invention, or lower.

It will be appreciated that according to a more preferred embodiment the process of the present invention may be carried out as a two step process in at least two separated reactor zones, in which two different synthesis gas streams are introduced, containing excess carbon monoxide and excess hydrogen respectively ($H_2/CO$ ratio varying from 0.5 to 1.9 and more preferably 1.0-1.8 and $H_2/CO$ ratio varying from 2.5 to 4.5 and more preferably from 2.9 to 3.5) compared to the stoichiometric consumption ratio
($H_2 CO = 2$).

In one of the zones a formate ester is formed (carbonylation step) e.g. methyl formate in the liquid phase using the catalyst system of the present invention in a low temperature process and using a feed gas, which is relatively rich in carbon monoxide, while in the other reaction zone this formate is hydrogenated in the liquid phase using the same catalyst composition and using a feed gas, which is relatively rich in hydrogen. The catalyst solution or slurry is circulated between the two reaction vessels.

In case a single synthesis gas stream is to be converted to methanol, a synthesis gas stream of higher $H_2/CO$ ratio is obtained by partial conversion of the first mentioned stream to form mainly the formate ester. The unconverted, hydrogen enriched residual gas stream from the first zone may be used to hydrogenate this formate ester in the second zone.

According to a more preferred embodiment these two feed streams are derived from natural gas, which is converted by partial oxidation to synthesis gas, relatively rich in carbon monoxide, and by steam reforming to a gas relatively rich in hydrogen.

Although it might initially seem advantageous to carry out the two consecutive reaction steps, i.e.

a) reaction of a carbon monoxide containing gas with an alcoholate to form a formate ester, e.g. methyl formate from alkali methanolate,

b) hydrogenation/hydrogenolysis of the formate ester to form methanol and the alcohol from the original alcoholate (preferably also methanol),
in a single reactor being the most simple and straight forward way, the following negative features of such single step process may be appreciated:

(1) Formation of formate is a rapid reaction, but is limited by thermodynamic equilibrium, while hydrogenation of formate on the other hand is only limited by kinetics. Although the equilibrium limitation of alcohol carbonylation was found to be lifted by consecutive removal of formate, the steady state formate concentration has appeared to be relatively low since the conditions are governed to a large extent by the requirements of the second step.

(2) When a stoichiometric hydrogen/carbon monoxide synthesis gas mixture is converted at high methanol yield, the methanol concentration in the liquid catalyst phase will be found to be relatively high under steady state conditions, even when originally a catalyst with a higher alcohol and/or alcoholate component has been applied.

It has been found now, that methanol is a less suitable alcohol component than an optimally selected one as far as the reaction is concerned with reference to reaction rates, mass transfer, gas solubilisation etc.

Moreover, at high methanol concentrations methylformate will inevitably be formed in competition with other formates.

Since methylformate is relatively volatile, it will be a substantial by-product of the methanol produced unless the steady state formate concentration can be kept deliberately low, which is not inductive to a high hydrogenation rate. Possible methylformate as byproduct can be reconverted into methanol.

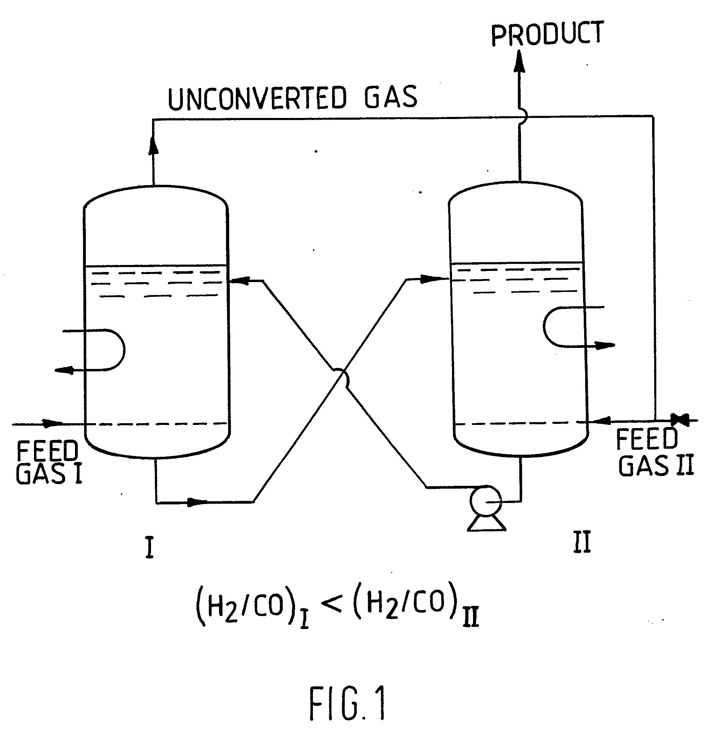

The hereinbefore described embodiment may be more particularly be carried out in a reaction equipment according to figure 1.

One of the individually produced gas mixtures relatively rich in CO is introduced in the left reactor where carbonylation is the predominant reaction. The carbonylation rate as well as equilibrium are favoured by the relatively high CO partial pressure (i.e. more than in a stoichiometric mixture usually applied).

The ester hydrogenation step (b) may predominantly take place in the right hand reactor. Herein the reaction rate benefits from the high hydrogen partial pressure and the relatively low CO pressure.

At a substantial conversion of the low $H_2/CO$ gas in the left reactor, there will be a net formate production with a $H_2/CO$ consumption ratio less than 2. In the right reactor, substantial conversion of the high $H_2/CO$ gas implies a net consumption of formate. Formate production and consumption may accurately balanced by control of the circulation of liquid catalyst system.

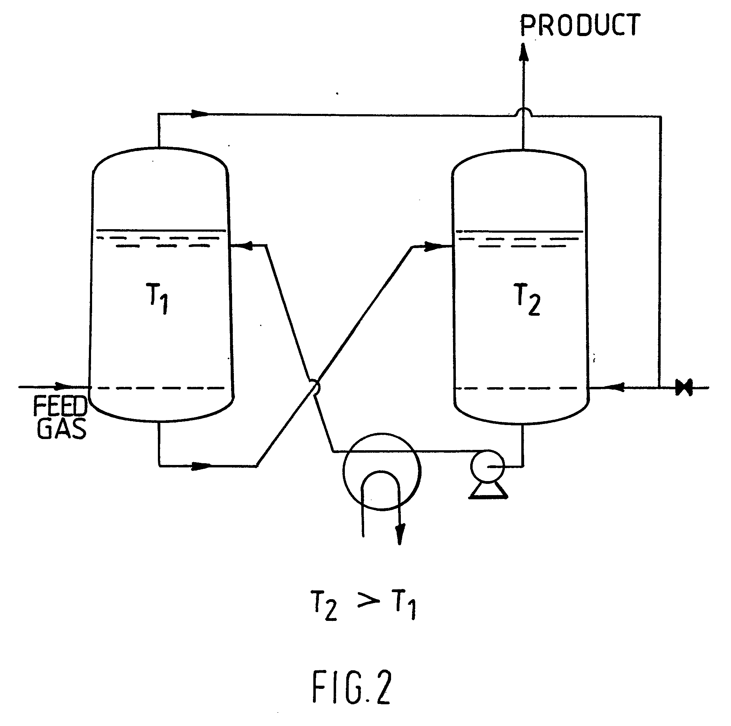

According to a more preferred embodiment the carbonylation reaction step is carried out at relatively low temperature (from 30-70 °C) in the left reactor, taking into account that this reaction step is a fast but equilibrium limited reaction, while the second hydrogenation reaction step is carried out at relatively higher temperature (from 70-120 °C) in the right hand reactor (reference is made to the figure 2), taking into account that the ester hydrogenation is not limited by equilibrium but by kinetics. Such a temperature difference as indicated hereinbefore, will be reached automatically, if the total reaction heat is removed from the circulating liquid catalyst system.

It will be appreciated that the before described embodiment offers a greater degree of flexibility than a single stage process, which leads to an overall better performance, e.g. a lower total reactor volume, while also the problem of methanol build up and by-product methyl formate will be minimized.

Moreover, this embodiment can certainly not be regarded as obvious to a skilled person who might only be inclined to look for improved single stage processes, due to the general conception that separate reaction vessels would mean a disadvantage.

The following examples further illustrate the invention, without however restricting the scope thereof to these particular embodiments.

All experiments were carried out in a 300 ml magnetically stirred Hastelloy C (Hastelloy is a trade name) autoclave. The reaction mixtures obtained were analyzed by means of gas-liquid chromatography.

Nickel formate was used which had been preheated under vacuum at 150 °C during 16 hours.

## Example 1

The autoclave was charged under a nitrogen atmosphere with diglyme (50 ml), nickel formate (10 mmol) and heated to a temperature of 45 °C under stirring and kept this temperature for 0.5 h.. Then a solution of 60 mmol potassium tert-butylate in 50 ml diglyme was added. The autoclave was sealed and methyl formate (10 ml) was added, giving pressure rise of 5 bar.

A mixture of carbon monoxide and hydrogen was introduced into the autoclave until a partial CO pressure of 15 bar and a partial $H_2$ pressure of 30 bar was reached.

The autoclave was further heated to a temperature of 80 °C and kept 5 hours at this temperature. The pressure was kept at a value between 30 and 60 bar by intermittent introduction of additional carbon monoxide and hydrogen up to pressure increases of 8 bar and 24 bar, of 7 bar and 21 bar, and three times of 8 bar and 24 bar respectively after 5 min, 25 min, 55 min, 1 hr and 25 min and 2 hr and 25 min respectively.

After termination of the reaction, 15.1 g methanol and 3.3 g methyl formate had been obtained.

## Example 2

In about the same way as described under example 1, an experiment was carried out, with the difference that instead of 10 ml methyl formate 5 g dimethyl oxalate was applied.

The pressure was kept in the range from 30-60 bar by continue addition of hydrogen and carbon monoxide in a 3:1 ratio.

After a reaction time of 5 hours at 80 °C, 6.2 g methanol and 6.3 g methyl formate had been obtained.

## Example 3

In about the same way as described under example 1, an experiment was carried out, with the difference that instead of 10 ml methyl formate 10 ml ethyl formate was used.

The pressure was kept in the range from 30-60 bar by continue addition of hydrogen and carbon monoxide in a 3:1 ratio.

After a reaction time of 2 hours at 80 °C 4.6 g ethanol and 8.9 g methanol had been obtained accompanied by traces of methyl formate and ethyl formate.

Comparative Example

If instead of 10 ml methyl formate 10 ml methyl proprionate is used, no reaction could be detected at all by means of $CO/H_2$ consumption after 5 hours at 80 °C.

**Claims**

1. A process for the production of methanol comprising contacting a gaseous mixture comprising carbon monoxide and hydrogen with a catalytic system, obtainable by combination of:
component (a): a nickel salt,
component (b): an in situ alcohol providing agent which originates from an external source, and
component (c): an alcoholate derived from an alkali metal or from an alkaline earth metal,
and activating the combined components.

2. A process as claimed in claim 1, characterized in that as component (a) nickel formate, nickel acetate, nickel oxalate or nickel tosylate is used.

3. A process as claimed in claim 1 or 2, characterized in that as component (b) alkyl formate, alkyl oxalate or alkyl carbonate or mixtures thereof with 1-10 carbon atoms in the alkyl group are used.

4. A process as claimed in claim 3, characterized in that methyl formate is used.

5. A process as claimed in any one of the claims 1-4, characterized in that as component (c) sodium or potassium alkoxides are used, preferably potassium tert-butoxide.

6. A process as claimed in any one of the claims 1-5, characterized in that it is carried out at a temperature between 30° and 120 °C, and a pressure between 5 and 100 bar.

7. A process as claimed in any one of the claims 1-6, characterized in that it is carried out in an organic diluent.

8. A process as claimed in any one of claims 1-7, characterized in that ethers are used as diluent, preferably diglyme.

9. A process as claimed in any one of the claims 1-8, characterized in that the mole ratio between components (a) and (b) is from 1:0.2 to 1:20, preferably from 1:3 to 1:8.

10. A process for the production of methanol according to claims 1-9, characterized in that it comprises:

a) a reaction step of a gas mixture having a higher partial pressure of carbon monoxide as compared to the stoichiometric synthesis gas ($H_2/CO$ ratio = 0.5-1.9) and obtained by partial oxidation of natural gas, to form a formate ester (carbonylation step),

b) hydrogenation/hydrogenolysis of the formate ester to form methanol and an alcohol from the original alcoholate, using a relatively hydrogen rich gas mixture of carbon monoxide and hydrogen wherein the hydrogen partial pressure is higher than in stoichiometric synthesis gas ($H_2/CO$ ratio = 2.5-4.5) and obtained by steam reforming of natural gas in at least two separated, interconnected reaction zones, between which the catalyst system is circulated.

11. A process as claimed in claim 10, characterized in that the carbonylation step is carried out at a temperature of from 30-70 °C and the hydrogenation step is carried out at a temperature of from 70-120 °C.

12. A catalyst system for the production of methanol obtainable by combination of:
component (a): a nickel salt,
component (b): an in situ alcohol providing agent, which originates from an external source, and
component (c): an alcoholate derived from an alkali metal or from an alkaline earth metal, and activating the combined components.

13. A catalyst system according to claim 12, characterized in that as component (a) nickel formate, nickel acetate, nickel oxalate or nickel tosylate is included.

14. A catalyst system according to claim 12 or 13, characterized in that as component (b) an alkyl formate, alkyl oxalate or alkyl carbonate with 1-10 carbon atoms in the alkyl group is included.

15. A catalyst system according to any one of claims 12-14, characterized in that as component (b) methyl formate or methyl oxalate or mixtures thereof are included.

16. A catalyst system according to any one of claims 12-15, characterized in that potassium tert-butoxide is included.

17. A catalyst system according to any one of claims 12-16, characterized in that the molar ratio of component (a) to component (b) is from 1:0.2 to 1:20, preferably from 1:3 to 1:8.

18. A process for the production of methanol as defined in claim 1, substantially as described hereinbefore, in particular with reference to the examples.

19. A catalyst system for the production of methanol as defined in claim 10, substantially as described hereinbefore with reference to the examples.

20. Methanol obtained by the process according to any one of the claims 1-11 and 18.

$$(H_2/CO)_I < (H_2/CO)_{II}$$

FIG.1

PRODUCT

FEED
GAS

$T_1$

$T_2$

$T_2 > T_1$

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | PATENT ABSTRACTS OF JAPAN, vol. 6, no. 57 (C-98)[935], 14th April 1982; & JP-A-56 169 634 (MITSUI SEKIYU KAGAKU KOGYO K.K.) 26-12-1981 * Abstract * | 1,5,12, 16 | C 07 C 31/04 C 07 C 29/15 C 07 C 29/136 C 07 C 69/04 C 07 C 67/36 B 01 J 31/04 |
| Y | US-A-4 613 623 (D. MAHAJAN) * Column 2, lines 16-59; columns 2-3, example 1; column 4, claims * | 1,6,7,8 ,12,13 | |
| D,Y | US-A-4 619 946 (R.S. SAPIENZA) * Column 1, line 59 - column 2, line 14; column 6, claims * | 1,6,12, 13 | |

TECHNICAL FIELDS SEARCHED (Int. Cl.4)

C 07 C 29/00
C 07 C 31/00

The present search report has been drawn up for all claims

| Place of search | Date of completion of the search | Examiner |
|---|---|---|
| THE HAGUE | 17-11-1988 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)